# EUROPEAN PATENT APPLICATION

(11) **EP 4 442 824 A1**
(43) Date of publication of application: **09.10.2024**
(21) Application number: 22901330.5
(22) Date of filing: 29.11.2022
(51) Int. Cl.: C12N 15/11, C12N 15/115, C12Q 1/6844

(54) **BINDING SUBSTANCE AND ANALYSIS METHOD**

(30) Priority: 02.12.2021 JP 2021196184
(71) Applicant: DENSO CORPORATION, Kariya-shi, Aichi 448-8661 (JP)
(72) Inventor: NIIMOTO, Mai, Kariya-shi, Aichi 448-8661 (JP); NUKAZUKA, Akira, Kariya-shi, Aichi 448-8661 (JP); ASANO, Mana, Kariya-shi, Aichi 448-8661 (JP); NAKAGAWA, Kazuhisa, Kariya-shi, Aichi 448-8661 (JP); HAYAKAWA, Kei, Kariya-shi, Aichi 448-8661 (JP)
(74) Representative: Kuhnen & Wacker Patent- und Rechtsanwaltsbüro PartG mbB
(86) International application number: PCT/JP2022/044042
(87) International publication number: WO 2023/100898

(57) **Abstract**

An analysis method detects a target substance (7) using a binding substance (1A). The binding substance includes: a single-stranded nucleic acid preparation (3) having activity to bind to the target substance; and a complementary nucleic acid (5) that forms a base pair (35) with a 3' terminal region (9) of the single-stranded nucleic acid preparation when the single-stranded nucleic acid preparation is not bound to the target substance, and that dissociates from the 3' terminal region when the single-stranded nucleic acid preparation is bound to the target substance.

## Description

### CROSS REFERENCE TO RELATED APPLICATION

This application is based on Japanese Patent Application No. 2021-196184 filed on December 2, 2021, the disclosure of which is incorporated herein by reference.

### TECHNICAL FIELD

The present disclosure relates to a binding substance and an analysis method.

### BACKGROUND ART

Conventionally, it discloses an analysis method for a target substance by using a fusion body. The fusion body includes a binding substance and a labeled substance. The binding substance has activity to bind to the target substance. The labeled substance induces an observable phenomenon.

In the analysis method for the target substance, a sample containing the target substance is mixed with the fusion bodies. Fusion bodies, which have not been bound to the target substance, are then separated. Thereafter, the occurrence of the phenomenon caused by fusion bodies bound to the target substance is detected. Patent documents 1 and 2 disclose the method for separating fusion bodies that are not bound to the target substance.

### PRIOR ART LITERATURES

### PATENT LITERATURE

Patent document 1 : JP 2019-020297 A
Patent document 2 : JP 2019-148556 A

### SUMMARY OF INVENTION

As a result of the detailed studies conducted by the inventors, the following issues have been found. When separating fusion bodies that are not bound to the target substance, various problems may arise. For example, the performance of a separation step increases the cost and time required for the analysis method. In addition, during the separation step, other substances may be introduced into the sample, reducing the accuracy of the analysis.

One aspect of the present disclosure provides a binding substance and analysis method that do not require any process of separating binding substances that are not bound to the target substance.

One aspect of the present disclosure provides a binding substance, which includes: a single-stranded nucleic acid preparation having activity to bind to a target substance; and a complementary nucleic acid that forms a base pair with a 3' terminal region of the single-stranded nucleic acid preparation when the single-stranded nucleic acid preparation is not bound to the target substance, and that dissociates from the 3' terminal region when the single-stranded nucleic acid preparation is bound to the target substance.

By using a binding substance according to one aspect of the present disclosure, the target substance can be analyzed without performing a process of separating a binding substance that is not bound to the target substance.

Another aspect of the present disclosure provides an analysis method for detecting a target substance using a binding substance. The binding substance includes: a single-stranded nucleic acid preparation having activity to bind to the target substance; and a complementary nucleic acid that forms a base pair with a 3' terminal region of the single-stranded nucleic acid preparation when the single-stranded nucleic acid preparation is not bound to the target substance, and that dissociates from the 3' terminal region when the single-stranded nucleic acid preparation is bound to the target substance.

The analysis method includes: mixing the binding substance and a sample containing the target substance; amplifying the single-stranded nucleic acid preparation; and detecting a phenomenon caused by the amplification of the single-stranded nucleic acid preparation.

According to the analysis method of another aspect of the present disclosure, the target substance can be analyzed without performing any process of separating a binding substance not bound to the target substance.

### BRIEF DESCRIPTION OF DRAWINGS

FIG. 1 is an explanatory diagram showing the structure of a first binding substance.
FIG. 2 is an explanatory diagram showing a hydroxyl group at the 3' position of a pentose.
FIG. 3 is an explanatory diagram showing the action of the first binding substance when the first binding substance coexists with a target substance.
FIG. 4 is an explanatory diagram showing a state in which a single-stranded nucleic acid preparation and a nucleic acid template form a complex.
FIG. 5 is an explanatory diagram showing the nucleic acid template and the first binding substance when the single-stranded nucleic acid preparation and its complementary nucleic acid form a double-stranded nucleic acid.
FIG. 6 is an explanatory diagram showing the structure and action of a second binding substance.
FIG. 7 is an explanatory diagram showing an analysis method.
FIG. 8 is an explanatory diagram showing a state of the first binding substance when a sample does not include the target substance.
FIG. 9 is a graph showing the degree of progress of rolling circle amplification.
FIG. 10 is a photograph showing the results of nucleic acid staining of an electrophoresis gel.

### DESCRIPTION OF EMBODIMENTS

Exemplary embodiments of the present disclosure will be described with reference to the drawings.

### 1. Binding substance 1

A binding substance 1 includes a single-stranded nucleic acid preparation 3 and a complementary nucleic acid 5. The single-stranded nucleic acid preparation 3 has an activity to bind to a target substance 7. The binding substance 1 has the activity to bind to the target substance 7 by including the single-stranded nucleic acid preparation 3. The target substance 7 is a substance to be analyzed. Examples of the target substance 7 include a protein, sugar, lipid, nucleic acid, or low molecular weight compound.

An example of the single-stranded nucleic acid preparation 3 is a nucleic acid aptamer. Examples of the nucleic acid aptamers include DNA aptamers and RNA aptamers. The single-stranded nucleic acid preparation 3 can be chemically synthesized, for example, by an in-vitro process. The number of bases in the single-stranded nucleic acid preparation 3 is, for example, 20 or more and 100 or less.

The single-stranded nucleic acid preparation 3 may be composed of, for example, a plurality of linked units, each unit being composed of a nucleic acid. The number of bases of each unit is preferably 20 or more and 100 or less. When the number of bases in each unit is 100 or less, even if the target substance 7 is located close to other substances, the single-stranded nucleic acid preparation 3 is less susceptible to interference from the other single-stranded nucleic acid preparation 3 and easily binds to the target substance 7.

The sequence of the single-stranded nucleic acid preparation 3 may be a DNA sequence, an RNA sequence, or a mixed sequence of DNA and RNA.

The sequence of the single-stranded nucleic acid preparation 3 may be a sequence further including modified nucleic acids or nucleic acid analogs, as long as the binding, hybridization, and elongation properties of the aptamer of the single-stranded nucleic acid preparation 3 are not impaired.

The complementary nucleic acid 5 forms a base pair 35 with a 3' terminal region 9 of the single-stranded nucleic acid preparation 3 when the single-stranded nucleic acid preparation 3 is not bound to the target substance 7. The complementary nucleic acid 5 dissociates from the 3' terminal region 9 when the single-stranded nucleic acid preparation 3 binds to the target substance 7.

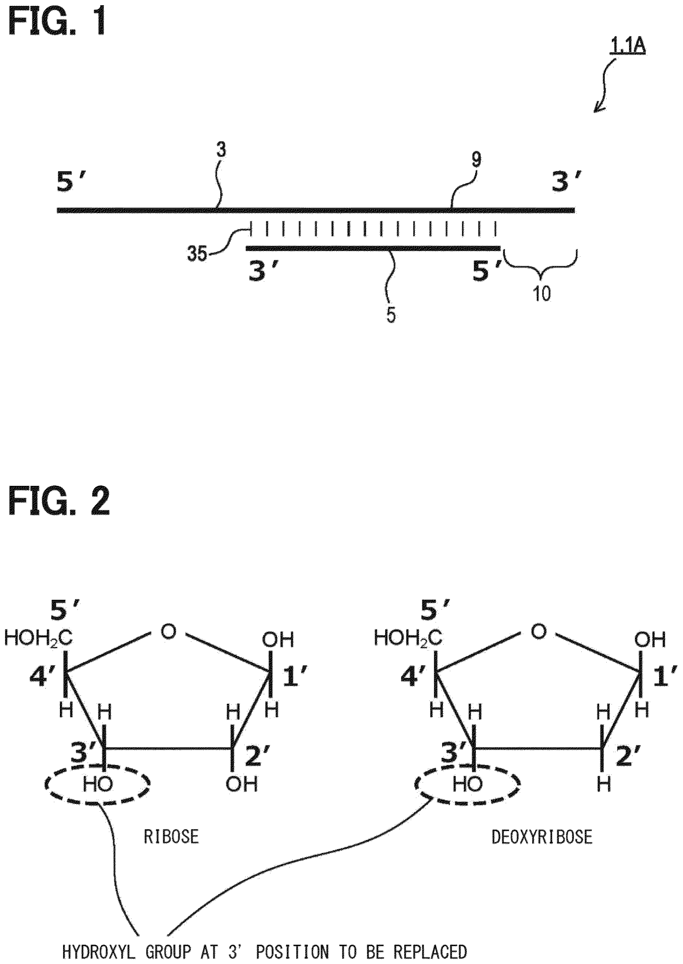

An example of the binding substance 1 is a first binding substance 1A shown in FIG. 1. The first binding substance 1A includes a single-stranded nucleic acid preparation 3 and a complementary nucleic acid 5. When the single-stranded nucleic acid preparation 3 is not bound to the target substance 7, the complementary nucleic acid 5 forms a double-stranded nucleic acid with the single-stranded nucleic acid preparation 3. The double-stranded nucleic acid corresponds to a double strand. When the single-stranded nucleic acid preparation 3 is not bound to the target substance 7, the complementary nucleic acid 5 forms a base pair 35 with the 3' terminal region 9 of the single-stranded nucleic acid preparation 3.

The 3' terminal region 9 is a region of the single-stranded nucleic acid preparation 3 on the 3' end side. The 3' terminal region 9 includes a base sequence that is complementary to a portion of the nucleic acid template 25 described below. The number of bases in the 3' terminal region 9 is preferably 10 or more and 30 or less. The GC content of the 3' terminal region 9 is preferably 30% or more and 70% or less.

The complementary nucleic acid 5 can be chemically synthesized, for example, by an in-vitro process. The number of bases in the complementary nucleic acid 5 is preferably 10 or more and 50 or less. The sequence of the complementary nucleic acid 5 may be a DNA sequence, an RNA sequence, or a mixed sequence of DNA and RNA.

The sequence of the complementary nucleic acid 5 may be a sequence further including modified nucleic acids or nucleic acid analogs, as long as the hybridization properties of the complementary nucleic acid 5 are not impaired. The complementary nucleic acid 5 hybridizes with the single-stranded nucleic acid preparation 3. The single-stranded nucleic acid preparation 3 has a blank region 10, for example, on the 3' end side. The blank region 10 is a region that does not form a base pair with the complementary nucleic acid 5. The number of bases in the blank region 10 is, for example, 0 or more and 15 or less. Preferably, 70% or more and 100% or less of the bases constituting the complementary nucleic acid 5 are those that are complementary to the single-stranded nucleic acid preparation 3.

The 3' most end of the complementary nucleic acid 5 is composed of a pentose such as ribose or deoxyribose, for example, shown in FIG. 2. The hydroxyl group at the 3' position of the pentose is replaced by a chemical substituent different from the hydroxyl group, for example. The chemical substituent different from the hydroxyl group is not particularly limited. Examples of chemical substituents different from the hydroxyl group include an azide group, an amine group, a biotin group, a phosphate group, a hydrogen group, fluorescence, and the like. The chemical substituents different from the hydroxyl group is a chemical substituent that does not form a phosphoester bond.

The first binding substance 1A can be synthesized by the process of hybridizing the single-stranded nucleic acid preparation 3 and the complementary nucleic acid 5. When synthesizing the first binding substance 1A, the ratio of the molar concentration of the complementary nucleic acid 5 to the molar concentration of the single-stranded nucleic acid preparation 3 (hereinafter referred to as the molar ratio R) is preferably 1 or more.

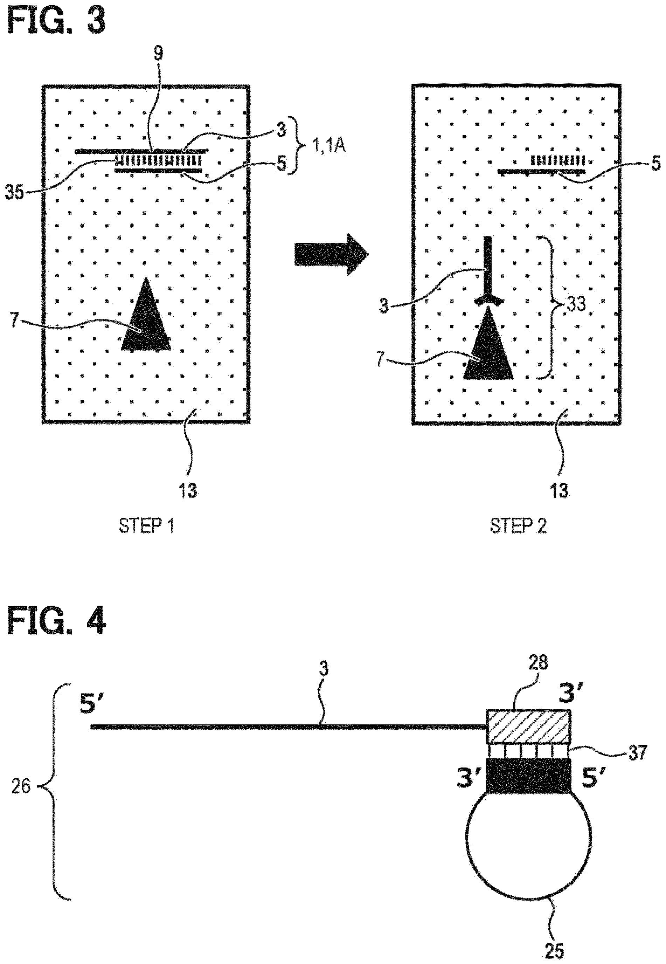

As shown in FIG. 3, when the first binding substance 1A coexists with the target substance 7, the single-stranded nucleic acid preparation 3 contained in the first binding substance 1A binds to the target substance 7. Accordingly, the complementary nucleic acid 5 dissociates from the single-stranded nucleic acid preparation 3. In a sample in which the target substance 7 is not present, the phenomenon of the dissociation of the complementary nucleic acid 5 from the single-stranded nucleic acid preparation 3 is unlikely to occur.

When the complementary nucleic acid 5 dissociates from the single-stranded nucleic acid preparation 3, the single-stranded nucleic acid preparation 3 can be amplified. The amplification of the single-stranded nucleic acid preparation 3 can be performed, for example, using the nucleic acid template 25 shown in FIG. 4.

The nucleic acid template 25 includes a base sequence complementary to a region 28 starting from the 3' most end of the single-stranded nucleic acid preparation 3. The nucleic acid template 25 and the single-stranded nucleic acid preparation 3 form a complex 26 by forming a base pair 37.

The total number of bases in the nucleic acid template 25 is 50 or more and 100 or less. Of all the bases of the nucleic acid template 25, the proportion of the bases that are complementary to the region 28 starting from the 3' end is preferably 70% or more and 100% or less, and more preferably 100%.

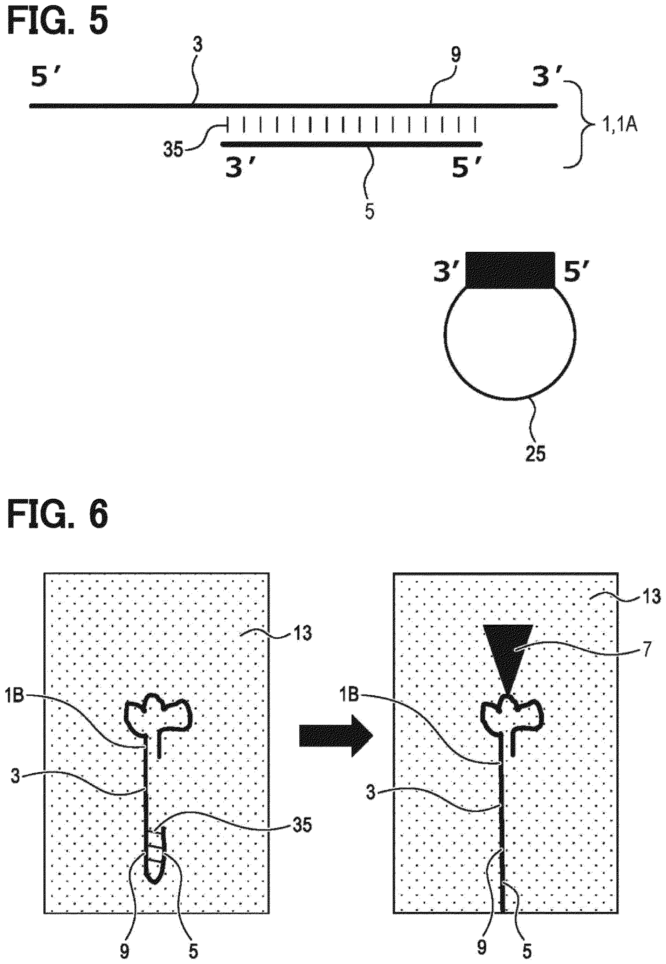

As shown in FIG. 5, when the single-stranded nucleic acid preparation 3 and the complementary nucleic acid 5 form a base pair 35, the nucleic acid template 25 cannot form the complex 26 with the single-stranded nucleic acid preparation 3. In other words, in a sample in which the target substance 7 is not present, the complex 26 between the nucleic acid template 25 and the single-stranded nucleic acid preparation 3 does not occur. When the single-stranded nucleic acid preparation 3 is not bound to the target substance 7, it is difficult to amplify.

The nucleic acid template 25 is, for example, a single-stranded cyclic nucleic acid. A single-stranded cyclic nucleic acid is, for example, single-stranded circular DNA. The single-stranded cyclic DNA is obtained by cyclizing the single-stranded linear DNA. Cyclization of single-stranded linear DNA can be performed using a DNA ligase such as CircLigase (Lucigen Corporation), CircLigase II (Lucigen Corporation), or T4 DNA Ligase (NEB Inc. and other companies).

An example of the binding substance 1 is a second binding substance 1B shown in FIG. 6. The second binding substance 1B includes the single-stranded nucleic acid preparation 3. When the single-stranded nucleic acid preparation 3 is not bound to the target substance 7, the single-stranded nucleic acid preparation 3 takes a folded structure in its molecule. When the single-stranded nucleic acid preparation 3 takes the folded structure in its molecule, the 3' terminal region 9 faces the complementary nucleic acid 5. The complementary nucleic acid 5 is part of the single-stranded nucleic acid preparation 3. When the single-stranded nucleic acid preparation 3 is folded, the complementary nucleic acid 5 and the 3' terminal region 9 facing thereto form the base pair 35.

When the complementary nucleic acid 5 forms base pair 35 with the 3' terminal region 9, the nucleic acid template 25 cannot form a complex 26 with the single-stranded nucleic acid preparation 3. When the single-stranded nucleic acid preparation 3 is not bound to the target substance 7, it is difficult to amplify.

When the single-stranded nucleic acid preparation 3 binds to the target substance 7, the complementary nucleic acid 5 dissociates from the 3' terminal region 9. When the complementary nucleic acid 5 dissociates from the 3' terminal region 9, the terminal region 9 can form the complex 26 with the nucleic acid template 25. Thus, when the single-stranded nucleic acid preparation 3 binds to the target substance 7, the single-stranded nucleic acid preparation 3 is easily amplified.

### 2. Analysis method

In the analysis method of the present disclosure, the binding substance 1 is used to detect the target substance 7. The binding substance 1 is the binding substance 1 described in the section "1. Binding Substance 1" above.

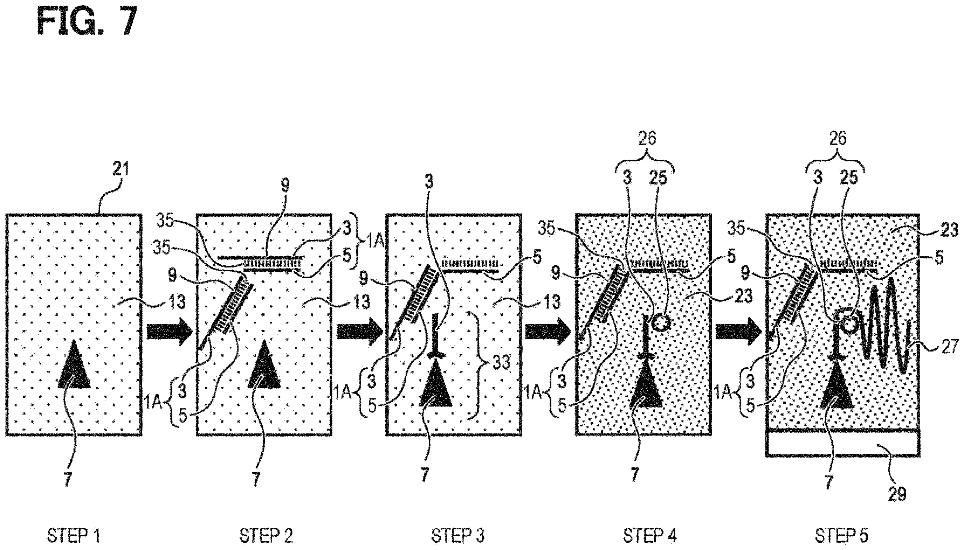

The analysis method for analyzing the sample 21 containing the target substance 7 using the first binding substance 1A is shown in FIG. 7. As shown in STEP 1 of FIG. 7, a sample 21 is prepared. The sample 21 contains the target substance 7 and a reaction solution 13 for conjugate formation, for example. The target substance 7 is present in the reaction solution 13 for conjugate formation.

Next, as shown in STEP 2 of FIG. 7, the first binding substance 1A and the sample 21 are mixed. The first binding substance 1A has the activity to bind to the target substance 7.

At this time, as shown in STEP 3 of FIG. 7, at least a portion of the first binding substance 1A binds to the target substance 7 to form a conjugate 33. In more detail, the single-stranded nucleic acid preparation 3 included in at least a portion of the first binding substance 1A binds to the target substance 7.

In the first binding substance 1A bound to the target substance 7, the complementary nucleic acid 5 dissociates from the single-stranded nucleic acid preparation 3, bringing the 3' terminal region 9 into a state of not forming the base pair 35. The single-stranded nucleic acid preparation 3 included in the first binding substance 1A bound to the target substance 7 becomes easy to amplify.

When a first binding substance 1A that is not bound to the target substance 7 is present in the sample 21, the single-stranded nucleic acid preparation 3 contained in the first binding substance 1A forms the base pair 35 with the complementary nucleic acid 5 and becomes difficult to amplify.

Next, as shown in STEP 4 of FIG. 7, a reaction solution 23 for nucleic acid amplification is added in place of the reaction solution 13 for conjugate formation. The reaction solution 23 for nucleic acid amplification contains known components of the strand displacement DNA synthetase, nucleic acid template 25, and reaction solution for nucleic acid amplification. Examples of the strand displacement DNA synthetase include a phi29 polymerase, a Vent DNA polymerase, a Bst DNA polymerase, and the like. The strand displacement DNA synthetase corresponds to a nucleic acid amplifying enzyme.

The composition of the reaction solution 23 for nucleic acid amplification can be adjusted as appropriate according to the phenomenon to be detected and the like. The phenomenon is one caused by nucleic acid amplification. The nucleic acid amplification is the amplification of the single-stranded nucleic acid preparation 3. For example, the molar concentration or pH of a buffer solution in the reaction solution 23 for nucleic acid amplification can be adjusted. After adding the reaction solution 23 for nucleic acid amplification, the target substance 7 and the first binding substance 1A are present in the reaction solution 23 for nucleic acid amplification.

Next, as shown in STEP 5 of FIG. 7, a process of amplifying the single-stranded nucleic acid preparation 3 is performed. The single-stranded nucleic acid preparation 3 included in the first binding substance 1A bound to the target substance 7 is amplified to generate an amplified nucleic acid 27. When a first binding substance 1A that is not bound to the target substance 7 is present in the reaction solution 23 for nucleic acid amplification, the single-stranded nucleic acid preparation 3 contained in the first binding substance 1A forms the base pair 35 with the complementary nucleic acid 5 and becomes difficult to amplify.

The amplification of the single-stranded nucleic acid preparation 3 is as described below, for example. As shown in FIG. 4, the single-stranded nucleic acid preparation 3 and the nucleic acid template 25 completely form the base pair 37 by the action of a sequence which is included in the nucleic acid template 25 and is complementary to the region 28 starting from the 3' most end, thereby forming the complex 26. Using the complex 26 as a starting point, the nucleic acid amplification occurs by the action of the nucleic acid amplifying enzyme.

Isothermal nucleic acid amplification is preferred as the nucleic acid amplification. Examples of isothermal nucleic acid amplification include the rolling circle amplification method (RCA), loop-mediated isothermal amplification method (LAMP), whole genome amplification method (WGA), multiple displacement amplification method (MDA), nicking endonuclease amplification reaction method (NEAR), and the like.

The isothermal nucleic acid amplification does not require temperature cycles such as temperature rise and fall, and its reaction proceeds at a constant temperature, making it easier to apply to simple detection methods, compared to a polymerase chain reaction (PCR) which requires temperature cycles such as temperature rise and fall.

Next, in STEP 5 of FIG. 7, the phenomenon caused by the amplification of the single-stranded nucleic acid preparation 3 is detected. Examples of the phenomenon include at least one of (a) coloration, luminescence, or fluorescence obtained by using a detection reagent that binds to the amplified single-stranded nucleic acid preparation 3, (b) generation of hydrogen ions associated with the incorporation of nucleotides through the amplification of the single-stranded nucleic acid preparation 3, and (c) generation of a pyrophosphoric acid associated with the incorporation of nucleotides through the amplification of the single-stranded nucleic acid preparation 3.

The reaction solution 23 for nucleic acid amplification contains, for example, a nucleic acid detection reagent. Examples of nucleic acid detection reagents include SYBR Green I, SYBR Green II, and the like. When the reaction solution 23 for nucleic acid amplification contains the nucleic acid detection reagent, the reaction solution 23 for nucleic acid amplification is excited by light at a specific wavelength and emits fluorescence when the nucleic acid amplification occurs. The fluorescence corresponds to the phenomenon caused by the amplification of the single-stranded nucleic acid preparation 3. The intensity of the fluorescence becomes higher as the amount of the single-stranded nucleic acid preparation 3 bound to the target substance 7 increases. The intensity of the fluorescence becomes higher as the amount of the target substance 7 contained in the sample 21 increases.

The intensity of fluorescence at the specific wavelength is increased in parallel with the nucleic acid amplification by using, for example, an intercalating fluorescent dye or minor groove luminescent dye. The fluorescence at the specific wavelength corresponds to the phenomenon caused by the amplification of the single-stranded nucleic acid preparation 3. The intensity of the fluorescence at the specific wavelength becomes higher as the amount of the single-stranded nucleic acid preparation 3 bound to the target substance 7 increases. The intensity of the fluorescence with the specified wavelength becomes higher as the amount of the target substance 7 contained in the sample 21 increases.

In the reaction solution 23 for nucleic acid amplification, nucleotides are incorporated into the amplified nucleic acids 27 as the nucleic acid amplification progresses, thus generating hydrogen ions. The amount of generated hydrogen ions is proportional to the amount of nucleotides incorporated. The pH of the reaction solution 23 for nucleic acid amplification decreases due to the generation of hydrogen ions. The generation of hydrogen ions and the decrease in pH correspond to the phenomena caused by the amplification of the single-stranded nucleic acid preparation 3. The amount of decrease in pH becomes greater as the amount of single-stranded nucleic acid preparation 3 bound to the target substance 7 increases. The amount of decrease in pH becomes greater as the amount of the target substance 7 contained in the sample 21 increases.

In the reaction solution 23 for nucleic acid amplification, nucleotides are incorporated into the amplified nucleic acids 27 as the nucleic acid amplification progresses, thus generating pyrophosphoric acid. When a group of enzymes that catalyze the reaction cascade driven by a pyrophosphoric acid is contained in the reaction solution 23 for nucleic acid amplification, the luminescence and change in oxidation-reduction potential occur due to the reaction cascade driven by the generated pyrophosphoric acid. The generation of pyrophosphoric acid, luminescence, and change in oxidation-reduction potential correspond to the phenomena that are caused by the amplification of the single-stranded nucleic acid preparation 3. The extents of luminescence and change in oxidation-reduction potential become greater as the amount of single-stranded nucleic acid preparation 3 bound to the target substance 7 increases. The extents of luminescence and change in oxidation-reduction potential become greater as the amount of the target substance 7 contained in the sample 21 increases.

In a case where the phenomenon is coloration, luminescence, or fluorescence, for example, the phenomenon can be detected using a light-receiving device. In a case where the phenomenon is the change in oxidation-reduction potential, the phenomenon can be detected using, for example, a potentiometer. In a case where the phenomenon is production, consumption, or absorption of hydrogen ions, the phenomenon can be detected using, for example, a pH meter.

The phenomena caused by nucleic acid amplification can be detected by a measuring instrument 29 shown in STEP 5 of FIG. 7. The measuring instrument 29 is, for example, a light-receiving device, a pH meter, a potentiometer, or the like.

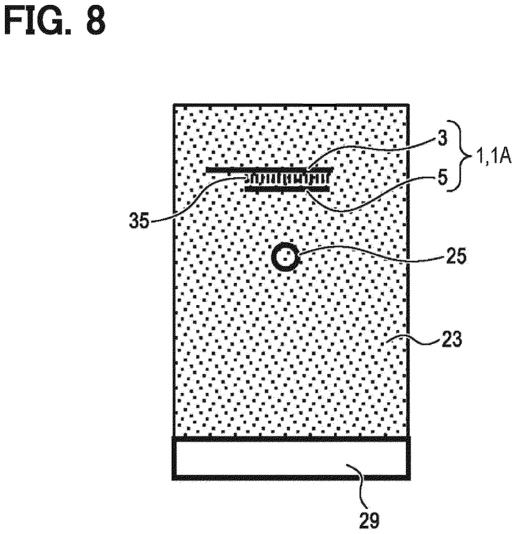

When the sample 21 does not contain the target substance 7, the single-stranded nucleic acid preparation 3 included in the first binding substance 1A forms the base pair 35 with the complementary nucleic acid 5, as shown in FIG. 8, even after the processes of STEPs 1 to 5 shown in FIG. 7 are performed. The single-stranded nucleic acid preparation 3 forming the base pair 35 is difficult to amplify. Therefore, when the sample 21 does not contain the target substance 7, the phenomenon caused by the amplification of the single-stranded nucleic acid preparation 3 is unlikely to occur even after the processes of STEPs 1 to 5 shown in FIG. 7 are performed.

When the nucleic acid template 25 is single-stranded cyclic DNA, the nucleic acid amplification using rolling circle amplification is caused by the action of the strand displacement DNA synthetase.

The nucleic acid amplification using the rolling circle amplification can be performed under isothermal conditions. When phi29 polymerase is used as the strand displacement DNA synthetase, the reaction of the nucleic acid amplification preferably proceeds at a constant temperature of 30°C or higher and 40°C or lower. The isothermal nucleic acid amplification generates an amplified nucleic acid 27.

When the pH meter is used as the measuring instrument 29 to detect the phenomenon caused by isothermal nucleic acid amplification, the reaction solution 23 for nucleic acid amplification used for isothermal amplification of the single-stranded nucleic acid preparation 3 by the rolling circle amplification preferably contains a buffer solution at a final concentration of 0 mM or more and 10 mM or less. An example of the buffer solution is a Tris (tris) buffer solution. In a case where isothermal nucleic acid amplification of the single-stranded nucleic acid preparation 3 is performed through the rolling circle amplification, the pH of the reaction solution 23 for nucleic acid amplification is preferably 7.0 or higher and 9.0 or lower. When the composition and pH of the reaction solution 23 for nucleic acid amplification are set as described above, changes in pH of the reaction solution 23 for nucleic acid amplification can be detected. The change in pH corresponds to the phenomenon caused by the isothermal nucleic acid amplification of the single-stranded nucleic acid preparations 3.

### 3. Effects exhibited by binding substance 1 and analysis method

(1A) In the first binding substance 1A that is not bound to the target substance 7, the nucleic acid amplification and phenomenon are unlikely to occur. Thus, even if the first binding substance 1A that is not bound to the target substance 7 is present in the reaction solution 23 for nucleic acid amplification shown in STEPs 4 and 5 of FIG. 7, the first binding substance 1A not bound to the target substance 7 does not easily interfere with detection of the target substance 7. As a result, a process of separating the first binding substance 1A not bound to the target substance 7 may not be performed between STEP3 and STEP4 in FIG. 7.

(1B) The first binding substance 1A includes the complementary nucleic acid 5. Therefore, the first binding substance 1A is even more effective in terms of (1A) above.

(1C) In STEP 5 of FIG. 7, the first binding substance 1A bound to the target substance 7 causes the nucleic acid amplification and phenomenon, so that the target substance 7 can be detected based on the caused phenomenon.

(1D) In the analysis method of the present disclosure, the concentration of hydrogen ions in the reaction solution 23 for nucleic acid amplification changes, for example, as a result of the nucleic acid amplification. In the analysis method of the present disclosure, for example, the change in the concentration of hydrogen ions caused in the reaction solution 23 for nucleic acid amplification can be measured using the pH meter. In such a case, the target substance 7 can be detected based on the measurement results of the pH meter.

(1E) In the analysis method of the present disclosure, the concentration of pyrophosphoric acid in the reaction solution 23 for nucleic acid amplification changes, for example, as a result of the nucleic acid amplification. In the analysis method of the present disclosure, for example, a change in the concentration of pyrophosphoric acid caused in the reaction solution 23 for nucleic acid amplification can be measured based on a change in the amount of luminescence and a change in the oxidation-reduction potential. In such cases, the target substance 7 can be detected based on changes in luminescence and oxidation-reduction potential.

(1F) In the analysis method of the present disclosure, the coloration, luminescence, or fluorescence caused in the reaction solution 23 for nucleic acid amplification can be measured, for example, by using the intercalating luminescent dye or minor groove luminescent dye that is adsorbed onto the amplified nucleic acid 27. In the analysis method of the present disclosure, for example, the target substance 7 can be detected based on the measurement results of the coloration, luminescence, or fluorescence caused in the reaction solution 23 for nucleic acid amplification.

(1G) In the analysis method of the present disclosure, for example, only the binding substance 1 bound to the target substance 7 is subject to the nucleic acid amplification. In such a case, as the concentration of the target substance 7 is higher, the color intensity, luminescence intensity, fluorescence intensity, change in oxidation-reduction potential, or change in pH, which is caused in the reaction solution 23 for nucleic acid amplification, is increased. Therefore, according to the analysis method of the present disclosure, the concentration of the target substance 7 can be quantitatively analyzed immunometrically.

(1H) In the analysis method of the present disclosure, for example, the isothermal nucleic acid amplification is performed at an isothermal temperature. In a case where the isothermal nucleic acid amplification is performed at the isothermal temperature, the reaction proceeds at a constant temperature. In a case where the isothermal nucleic acid amplification is performed at the isothermal temperature, the analysis method of the present disclosure is applied to a simple detection method more easily than a polymerase chain reaction that requires a temperature cycle including temperature rise and fall.

### 4. Example

### (4-1) Synthesis of first binding substance 1A

The DNA sequence of SEQ ID NO: 1 was synthesized. The DNA sequence of SEQ ID NO: 1 corresponds to the single-stranded nucleic acid preparation 3. The DNA sequence of SEQ ID NO: 1 was a partially modified version of the DNA sequence described in Anal.Chem.2020, 92, 9895-9900 (hereafter referred to as Reference 1).

The DNA sequence of SEQ ID NO: 1 includes a DNA sequence of SEQ ID NO: 2. Reference 1 states that the DNA sequence of SEQ ID NO: 2 is identified as a DNA aptamer in which an RBD region in a spike glycoprotein of the novel coronavirus SARS-CoV-2 (hereafter referred to as the RBD) is the target substance 7.

The DNA sequence of SEQ ID NO: 1 includes a DNA sequence of SEQ ID NO: 3. The DNA sequence of SEQ ID NO: 3 is a DNA sequence that is complementary to the complementary nucleic acid 5 and corresponds to the 3' terminal region 9.

The DNA sequence of SEQ ID NO: 1 includes a DNA sequence of SEQ ID NO: 4 in the region 28 starting from the 3' most end. The DNA sequence of SEQ ID NO: 4 is a DNA sequence complementary to the nucleic acid template 25.

The synthesized single-stranded nucleic acid preparation 3 having the DNA sequence of SEQ ID NO: 1 was dissolved in pure water to prepare a single-stranded nucleic acid preparation solution. The final concentration of the single-stranded nucleic acid preparation 3 in the single-stranded nucleic acid preparation solution was 100 µM.

The complementary nucleic acid 5 having the DNA sequence of SEQ ID NO: 5 was synthesized. The 3' position hydroxyl group at the 3' most end of the complementary nucleic acid 5 was substituted with an amino group. After the substitution of the amino group, the complementary nucleic acid 5 was dissolved in pure water to prepare a complementary nucleic acid solution. The final concentration of the complementary nucleic acid 5 in the complementary nucleic acid solution was 100 µM.

A reaction solution containing a NaCl (sodium chloride)-Tris-EDTA buffer (buffer), the single-stranded nucleic acid preparation 3, the complementary nucleic acid 5, and pure water was prepared.

The final concentration of Tris was 10 mM. The final concentration of EDTA was 1 mM. The final concentration of NaCl was 50 mM. The pH of the NaCl-Tris-EDTA was 7.5. The final concentration of the single-stranded nucleic acid preparation 3 was 0.5 µM. The final concentration of the complementary nucleic acid 5 was 0 µM, 0.5 µM, 5 µM, or 50 µM.

That is, the reaction solution was classified into four types that differed in the final concentration of the complementary nucleic acid 5. When the molar concentration of the single-stranded nucleic acid preparation 3 in the reaction solution was 1, the molar concentration of the complementary nucleic acid 5 in the reaction solution was any one of 1, 10, and 100. In other words, the molar ratio R in the reaction solution was 0, 1, 10, or 100.

For each of the four types of reaction solutions, the reaction solution was heated at 95°C for 5 minutes, and then its temperature was lowered to 25°C at a constant rate over 60 minutes. As a result, when the molar ratio R was 1, 10, or 100, the single-stranded nucleic acid preparation 3 and the complementary nucleic acid 5 formed the base pair 35, yielding the first binding substance 1A. In the single-stranded nucleic acid preparation 3, the 3' terminal region 9 formed the base pair 35 with the complementary nucleic acid 5. In the first binding substance 1A, the single-stranded nucleic acid preparation 3 and the complementary nucleic acid 5 formed the base pair 35. When the molar ratio R was 0, the base pair 35 remained without forming any base pair 35.

### (4-2) Synthesis of nucleic acid template 25

The single-stranded linear DNA having the DNA sequence of SEQ ID NO: 6 was synthesized. Next, the 5' end of the single-stranded linear DNA was phosphorylation-modified. Then, the single-stranded linear DNA was cyclized by the action of CircLigase II to form single-stranded circular DNA. Then, the single-stranded linear DNA remaining without cyclization was decomposed by being treated with Exonuclease I, and the single-stranded cyclic DNA was purified and extracted. The purified and extracted single-stranded circular DNA was used as the nucleic acid template 25. The nucleic acid template 25 included a sequence complementary to the region 28 starting from the 3' most end of the single-stranded nucleic acid preparation 3. The sequence of the region 28 starting from the 3' most end of the single-stranded nucleic acid preparation 3 was a sequence of SEQ ID NO: 4.

### (4-3) Verification of amplification

It was verified whether the rolling circle amplification proceeded in the reaction solution for nucleic acid amplification containing the first binding substance 1A as follows.

The reaction solution 23 for nucleic acid amplification, containing a phi29 DNA polymerase (NEB Inc., M0269) and SYBR Green II (Takara Bio Inc., 5771A), was prepared. More specifically, the reaction solution 23 for nucleic acid amplification contained a 10x phi29 buffer (final concentration of 1x), a dNTP mix (final concentration of 0.2 mM), BSA (final concentration of 0.1 mg/mL), SYBR Green II (final concentration of 1x), a phi29 DNA polymerase (final concentration of 0.05 U/µL), the nucleic acid template 25 (final concentration of 100 nM), the product obtained by the process described in (4-1) above (final concentration of the single-stranded nucleic acid preparation 3 of 100 nM), and pure water. The volume of the reaction solution 23 for nucleic acid amplification was 25 µL.

The product of the process in (4-1) above was the product when the molar ratio R was 0, when the molar ratio R was 1, when the molar ratio R was 10, or when the molar ratio R was 100.

Using a real-time PCR analysis system (Bio-Rad Laboratories, Inc., CFX Connect (registered trademark)), the reaction solution 23 for nucleic acid amplification prepared above was incubated at 30°C for 3 hours. During the incubation, fluorescence kinetics of SYBR Green II were measured in real time.

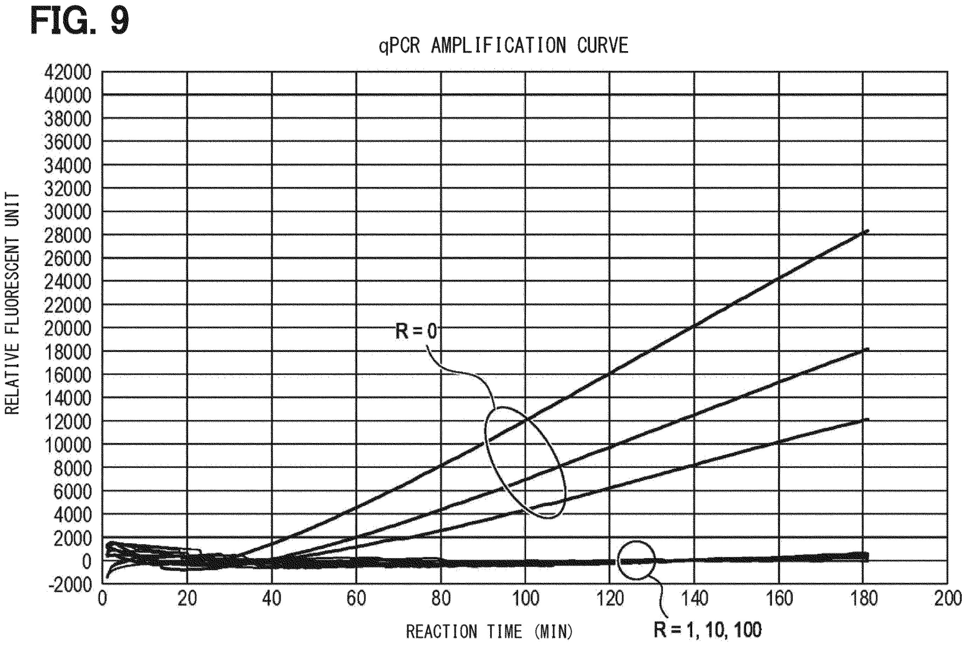

The measurement results are shown in FIG. 9. As shown in FIG. 9, an increase in fluorescence intensity over time was confirmed when the product of the process in (4-1) above was obtained under the condition where the molar ratio R was 0. On the other hand, as shown in FIG. 9, no increase in fluorescence intensity was confirmed when the product of the process in (4-1) above was obtained under the condition where the molar ratio R was 1, 10, or 100.

The measurement results indicate the following. When the product of the process in (4-1) above was obtained under the condition where the molar ratio R was 0, the product was a single-stranded nucleic acid preparation 3 that did not form the base pair 35. The single-stranded nucleic acid preparation 3 that did not form the base pair 35 formed a new base pair 37 with the nucleic acid template 25 to form the complex 26. Then, starting from the formation of the complex 26, rolling circle amplification occurred, and the nucleic acid was amplified. As a result, the phenomenon caused by the amplification of the nucleic acid was able to be observed.

When the product of the process in (4-1) above was obtained under the condition where the molar ratio R was 1, 10, or 100, the product was the first binding substance 1A. The single-stranded nucleic acid preparation 3 included in the first binding substance 1A formed the base pair 35 with the complementary nucleic acid 5. Thus, the nucleic acid template 25 was not able to form the complex 26 with the single-stranded nucleic acid preparation 3. As a result, no amplification of the nucleic acid occurred, and the phenomenon caused by the amplification of the nucleic acid was not able to be observed.

### (4-4) Verification of dissociation

It was verified whether or not the complementary nucleic acid 5 dissociated from the single-stranded nucleic acid preparation 3, along with the formation of a conjugate between the single-stranded nucleic acid preparation 3 and the target substance 7, when the target substance 7 and the first binding substance 1 coexist together. This verification was made in the following manner.

The first binding substance 1A was synthesized by the method described in "(4-1) Synthesis of first binding substance 1A" above. When synthesizing the first binding substance 1A, each of the molar concentration of the single-stranded nucleic acid preparation 3 in the reaction solution and the molar concentration of the complementary nucleic acid 5 in the reaction solution was 0.5 µM. In other words, the molar ratio R in the reaction solution was 1.

Spike S1-His Recombinant Protein (Sino Biological Inc., product number 40591-V08H) (hereafter referred to as S1) was used as the target substance 7. S1 is a protein containing an RBD in its amino acid sequence.

A reaction solution containing the first binding substance 1 (final concentration of 100 nM), S1 (final concentration of 0 nM, 25 nM, 50 nM, 100 nM, or 200 nM), and 1x PBS/T was prepared.

1x PBS/T is a phosphate buffer solution containing 0.05% (v/v) of surfactant Tween 20. The phosphate buffer solution contained 137 mM of NaCl, 8.1 mM of Na₂HPO₄, 2.7 mM of KCl, and 1.47 mM of KH₂PO₄. The reaction solution was incubated at 25°C over 30 minutes.

The incubation reaction products were separated by size using electrophoresis. A 4%-20% concentration gradient gel (Thermo Fisher Scientific Inc., EC62255BOX) was used as an electrophoresis gel. The electrophoresis gel and an electrophoresis tank (Thermo Fisher Scientific Inc., EI0001) were filled with a 1x TBE electrophoresis buffer. The TBE electrophoresis buffer contained 89 mM of Tris, 89 mM of boric acid, and 2 mM of EDTA.

5 µL of the reaction solution after the incubation was resuspended in 2 µL of electrophoresis sample buffer (Thermo Fisher Scientific Inc., LC6678) and 3 µL of pure water. 4 µL of the resuspended solution was subjected to electrophoresis. Here, 4 µL of this solution subjected to the electrophoresis contained 0.2 pmol of the first binding substance 1.

The electrophoresis control was subjected to electrophoresis in the same manner. The electrophoresis control contained 0.2 pmol of complementary nucleic acid 5.

The size maker was subjected to electrophoresis in the same manner. The size marker contains 27.2 ng of 50 bp-ladder (Promega Corporation, G452A).

A voltage of 180 volts was applied, and electrophoresis was performed over 40 minutes. The electrophoresis gel was then removed. The nucleic acids were then stained using SYBR Gold (Thermo Fisher Scientific Inc., S11494, final concentration of 1x). Next, the gel was photographed with a gel photography machine (Bio-Rad Laboratories, Inc., ChemiDoc MP).

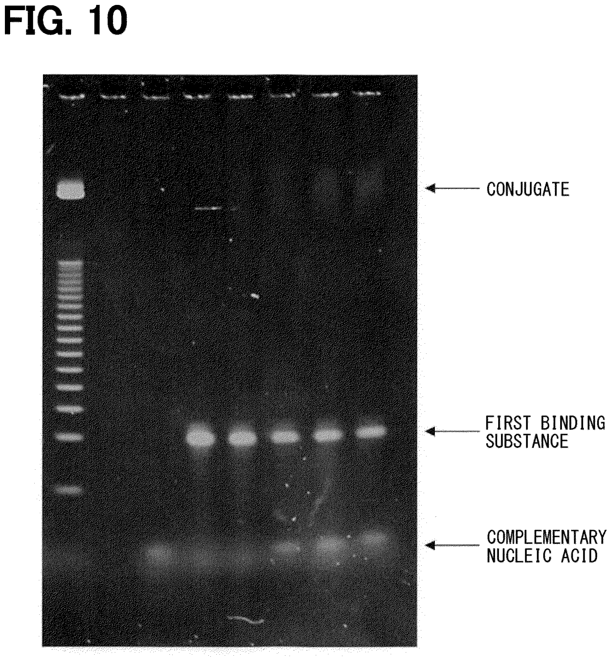

The results of nucleic acid staining using the electrophoresis gel are shown in FIG. 10. The composition of a solution used in each lane shown in FIG. 10 was as follows:
Lane 1: Size maker (50bp-ladder)
Lane 2: Solution containing only 100 nM of complementary nucleic acid 5.
Lane 3: Reaction solution containing 100 nM of the first binding substance 1 without containing S1.
Lane 4: Reaction solution containing 100 nM of the first binding substance 1 and 25 nM of S1.
Lane 5: Reaction solution containing 100 nM of the first binding substance 1 and 50 nM of S1.
Lane 6: Reaction solution containing 100 nM of the first binding substance 1 and 100 nM of S1.
Lane 7: Reaction solution containing 100 nM of the first binding substance 1 and 200 nM of S1.

In the lane where the reaction solution not containing S1 was electrophoresed, a signal located at the molecular weight of the complementary nucleic acid 5 was not confirmed. In the lane where the reaction solution containing S1 was electrophoresed, a signal located at the molecular weight of the complementary nucleic acid 5 was confirmed. The intensity of the signal located at the molecular weight of the complementary nucleic acid 5 became stronger as the concentration of S1 increased.

In the lane where the reaction solution containing S1 was electrophoresed, the intensity of the signal located at the molecular weight of the first binding substance 1A became weaker as the concentration of S1 increased. In the lane where the reaction solution containing S1 was electrophoresed, a conjugate 33 was obtained by binding the single-stranded nucleic acid preparation 3 to S1 and had a large apparent molecular weight, and a signal assumed to be derived from this conjugate 33 was confirmed. The intensity of the signal assumed to be derived from the conjugate 33 became higher as the concentration of S1 increased.

The results of the nucleic acid staining of the electrophoresis gel show the following. The first binding substance 1A had the activity to bind to S1 and form the conjugate 33. The complementary nucleic acid 5 dissociated from the single-stranded nucleic acid preparation 3, with the formation of the conjugate 33 as the starting point. In response, the single-stranded nucleic acid preparation 3 formed the conjugate 33 with S1.

### 5. Other Embodiments

The embodiments of the present disclosure have been described above, but the present disclosure is not limited to the embodiments described above and can be implemented with various variations.

(1) In the analysis method and example shown in FIG. 7, a second binding substance 1B may be used instead of the first binding substance 1A.

Nucleic acid amplification and phenomena are unlikely to occur in the second binding substance 1B, which was not bound to the target substance 7. Thus, even if the second binding substance 1B that is not bound to the target substance 7 is present in the reaction solution 23 for nucleic acid amplification shown in STEPs 4 and 5 of FIG. 7, it does not easily interfere with detection of the target substance 7. As a result, a process of separating the second binding substance 1B not bound to the target substance 7 may not be performed between STEP3 and STEP4 in FIG. 7.

In STEP 5 of FIG. 7, the second binding substance 1B, which was bound to the target substance 7, caused nucleic acid amplification and phenomena, so that the target substance 7 can be detected.

(2) A plurality of functions associated with one component in the above embodiments may be implemented by a plurality of components, or one function associated with one component may be implemented by a plurality of components. A plurality of functions associated with a plurality of components may be implemented by a single component, or a single function implemented by a plurality of components may be implemented by a single component. Some of the configurations of the above embodiments may be omitted. Also, at least a portion of the configuration of the above embodiments may be added or substituted for other configurations of the above embodiments.

(3) In addition to the analysis method described above, the present disclosure can also be implemented in various forms, such as producing methods for binding substances.

### [Technical ideas disclosed by the present specification]

### [Item 1]

A binding substance (1, 1A, 1B) comprising:
a single-stranded nucleic acid preparation (3) having activity to bind to a target substance (7); and
a complementary nucleic acid (5) that forms a base pair 35 with a 3' terminal region (9) of the single-stranded nucleic acid preparation when the single-stranded nucleic acid preparation is not bound to the target substance, and that dissociates from the 3' terminal region when the single-stranded nucleic acid preparation is bound to the target substance.

### [Item 2]

The binding substance according to Item 1, wherein
the complementary nucleic acid is a part of the single-stranded nucleic acid preparation, and
the single-stranded nucleic acid preparation has a folded structure in a molecule thereof, whereby the part of the single-stranded nucleic acid preparation forms the base pair with the 3' terminal region.

### [Item 3]

An analysis method for detecting a target substance (7) using a binding substance (1, 1A, 1B),
the binding substance comprising:
   a single-stranded nucleic acid preparation (3) having activity to bind to the target substance; and
   a complementary nucleic acid (5) that forms a base pair 35 with a 3' terminal region (9) of the single-stranded nucleic acid preparation when the single-stranded nucleic acid preparation is not bound to the target substance, and that dissociates from the 3' terminal region when the single-stranded nucleic acid preparation is bound to the target substance,
the analysis method comprising:
   mixing the binding substance and a sample (21) containing the target substance;
   amplifying the single-stranded nucleic acid preparation; and
   detecting a phenomenon caused by the amplification of the single-stranded nucleic acid preparation.

### [Item 4]

The analysis method according to Item 3, wherein
the phenomenon is at least one of (a) coloration, luminescence, or fluorescence obtained by using a detection reagent that binds to the amplified single-stranded nucleic acid preparation, (b) generation of hydrogen ions associated with incorporation of a nucleotide through the amplification of the single-stranded nucleic acid preparation, and (c) generation of a pyrophosphoric acid associated with the incorporation of a nucleotide through the amplification of the single-stranded nucleic acid preparation.

### [Item 5]

The analysis method according to Item 3 or 4, wherein
the phenomenon is detected using a light-receiving device (29) in a case where the phenomenon is coloration, luminescence, or fluorescence,
the phenomenon is detected using a potentiometer (29) in a case where the phenomenon is a change in oxidation-reduction potential, and
the phenomenon is detected using a pH meter (29) in a case where the phenomenon is production, consumption, or absorption of hydrogen ions.

### [Item 6]

The analysis method according to Items 3 to 5, wherein
the single-stranded nucleic acid preparation is amplified by an isothermal nucleic acid amplification method not requiring a temperature cycle.

### [Item 7]

The analysis method according to Items 3 to 6, further comprising:
forming a complex (26) by using a single-stranded cyclic nucleic acid (25) including a sequence complementary to the 3' terminal region and the single-stranded nucleic acid preparation, and
amplifying the single-stranded nucleic acid preparation with the complex serving as a starting point by a rolling circle amplification method using a reaction solution (23) for nucleic acid amplification.

### [Item 8]

The analysis method according to Items 3 to 6, wherein
the single-stranded nucleic acid preparation is amplified by a rolling circle amplification method,
a reaction solution (23) for nucleic acid amplification for amplifying the single-stranded nucleic acid preparation contains a Tris buffer solution at a final concentration of 0 mM or more and 10 mM or less,
a pH of the reaction solution for nucleic acid amplification is 7.0 or higher and 9.0 or lower, and
the phenomenon is detected using a pH meter (29).

### [Item 9]

The analysis method according to Items 3 to 8, wherein
the target substance is a protein, sugar, lipid, nucleic acid, or low molecular weight compound.

## Claims

1. A binding substance (1, 1A, 1B) comprising:
a single-stranded nucleic acid preparation (3) having activity to bind to a target substance (7); and
a complementary nucleic acid (5) that forms a base pair 35 with a 3' terminal region (9) of the single-stranded nucleic acid preparation when the single-stranded nucleic acid preparation is not bound to the target substance, and that dissociates from the 3' terminal region when the single-stranded nucleic acid preparation is bound to the target substance.

2. The binding substance according to claim 1, wherein
the complementary nucleic acid is a part of the single-stranded nucleic acid preparation, and
the single-stranded nucleic acid preparation has a folded structure in a molecule thereof, whereby the part of the single-stranded nucleic acid preparation forms the base pair with the 3' terminal region.

3. An analysis method for detecting a target substance (7) using a binding substance (1, 1A, 1B),
the binding substance comprising:
a single-stranded nucleic acid preparation (3) having activity to bind to the target substance; and
a complementary nucleic acid (5) that forms a base pair 35 with a 3' terminal region (9) of the single-stranded nucleic acid preparation when the single-stranded nucleic acid preparation is not bound to the target substance, and that dissociates from the 3' terminal region when the single-stranded nucleic acid preparation is bound to the target substance,
the analysis method comprising:
mixing the binding substance and a sample (21) containing the target substance;
amplifying the single-stranded nucleic acid preparation; and
detecting a phenomenon caused by the amplifying of the single-stranded nucleic acid preparation.

4. The analysis method according to claim 3, wherein
the phenomenon is at least one of (a) coloration, luminescence, or fluorescence obtained by using a detection reagent that binds to the amplified single-stranded nucleic acid preparation, (b) generation of hydrogen ions associated with incorporation of a nucleotide through the amplification of the single-stranded nucleic acid preparation, and (c) generation of a pyrophosphoric acid associated with the incorporation of a nucleotide through the amplification of the single-stranded nucleic acid preparation.

5. The analysis method according to claim 3 or 4, wherein
the phenomenon is detected using a light-receiving device (29) in a case where the phenomenon is coloration, luminescence, or fluorescence,
the phenomenon is detected using a potentiometer (29) in a case where the phenomenon is a change in oxidation-reduction potential, and
the phenomenon is detected using a pH meter (29) in a case where the phenomenon is production, consumption, or absorption of hydrogen ions.

6. The analysis method according to claim 3 or 4, wherein
the single-stranded nucleic acid preparation is amplified by an isothermal nucleic acid amplification method not requiring a temperature cycle.

7. The analysis method according to claim 3 or 4, further comprising:
forming a complex (26) by using a single-stranded cyclic nucleic acid (25) including a sequence complementary to the 3' terminal region and the single-stranded nucleic acid preparation, and
amplifying the single-stranded nucleic acid preparation with the complex serving as a starting point by a rolling circle amplification method using a reaction solution (23) for nucleic acid amplification.

8. The analysis method according to claim 3 or 4, wherein
the single-stranded nucleic acid preparation is amplified by a rolling circle amplification method,
a reaction solution (23) for nucleic acid amplification for amplifying the single-stranded nucleic acid preparation contains a Tris buffer solution at a final concentration of 0 mM or more and 10 mM or less,
a pH of the reaction solution for nucleic acid amplification is 7.0 or higher and 9.0 or lower, and
the phenomenon is detected using a pH meter (29).

9. The analysis method according to claim 3 or 4, wherein
the target substance is a protein, sugar, lipid, nucleic acid, or low molecular weight compound.
